# EUROPÄISCHE PATENTANMELDUNG

(11) **EP 1 493 432 A1**
(43) Veröffentlichungstag der Anmeldung: **05.01.2005**
(21) Anmeldenummer: 04017617.4
(22) Anmeldetag: 24.12.1993
(51) Int. Cl.: A61K 7/48, C07D 239/06

(54) **Verfahren zur in vivo Gewinnung von Inhaltsstoffen aus Zellen**

(30) Priorität: 31.12.1992 DE 4244580
(62) Teilanmeldung aus: 98121244.2
(71) Anmelder: MERCK PATENT GmbH, 64293 Darmstadt (DE)
(72) Erfinder: Galinski, Erwin, Dr., 48308 Senden (DE); Trüper, Hans, Prof. Dr., 53177 Bonn (DE); Sauer, Thomas, 53121 Bonn (DE)

(57) **Zusammenfassung**

Es wird ein Verfahren beschrieben zur in vivo Gewinnung von Inhaltsstoffen aus Zellen, wobei die betreffenden Zellen von einem ersten Zustand, in dem quasi-stationäre Bedingungen zwischen Zelle und umgebendem Medium existieren, plötzlich in einem zweiten Zustand wechseln, wodurch die Zellen gezwungen werden, durch Abgabe von Zellinhaltsstoffen in die Umgebung zu reagieren, um eine Anpassung der Zelle an die Bedingungen des zweiten Zustandes zu erreichen.

## Beschreibung

Gegenstand der vorliegenden Erfindung ist die Verwendung von Hydroxyectoin und/oder Ectoin in der Kosmetik.

Aus E. A. Galinski, K. Lippert, NATO ASI Ser., Ser. A 1991,201 (Gen. Appl. Aspects Halophilic Microorg.) 351-8 ist das vorkommen von Ectoinen in halophilen Mikroorganismen, die Struktur der Verbindung sowie ihre Extraktion aus den halophilen Mikroorganismen bekannt. Weiter ist aus der Publikation die Verwendung von Ectoinen zur Stabilisierung von labilen Enzymen bekannt. Dabei wird die Kryoprotektion von Lactatdehydrogenase durch Ectoin untersucht.

In E. A. Galinski, K. Lippert, Appl. Microbiol. Biotechnol. 1992, 37(1 ), 61-5 wird der Effekt der kompatiblen Solute Ectoin und Hydroxyectoin bei der Stabilisierung der empfindlichen Enzyme Lactatdehydrogenase und Phosphofructokinase gegen Hitze, Kälte und Austrocknung untersucht.

Verbindungen mit dem Strukturelement Formel sind beispielsweise Naturstoffe, die aus entsprechenden Kulturen isoliert werden können. Verbindungen, die das Strukturelement

R¹-N=C(R²)-NHR³ I

enthalten, können in protonierter Form mit den prototropen Strukturen (II) und (III) wiedergegeben werden,

R¹⁺-N=C(R²)-NHR³ II ↔ R¹-N=C(R²)-NHR³ I

Dabei sind R¹ und/oder R³ Alkyl-, Cycloalkyl oder mit Heteroatomen substituierte oder funktionalisierte Derivate, wobei die Derivatisierung mit Hydroxy-, Sulfonsäure-, Carbonsäurederivate, wie Amide, Ester usw., Carbonyl, Ether, Alkoxy- und andere Hydroxylgruppenderivate erfolgt ist und wobei R¹ und R³ zusammen mit dem Strukturelement gemäß Formel I einen Ring bilden können
sowie R² Wasserstoff ist oder eine der für R¹, R³ genannte Bedeutungen besitzt.

Die Verbindungen können zur Stabilisierung von Enzymen und anderen Biomolekülen in wässrigen Lösungen und organischen Lösungsmittel sowie zur Stabilisierung gegen denaturierende Bedingungen verwendet werden.

Als Biomoleküle kommen neben Enzymen beispielsweise Proteine oder weitere Biopolymere in Frage. Als denaturierende Reagenzien sind Salze, extreme pH-Werte, Tenside, Harnstoff, Guanidiniumchlorid und andere Verbindungen zu nennen. Die Verwendung der genannten Stoffe mit dem Strukturelement I erlaubt die Erhaltung der komplexen Struktur der Biomoleküle selbst unter den genannten Bedingungen.

Vorzugsweise werden Verbindungen eingesetzt, die das Strukturelement I in einem 6- bis 8-gliedrigen System enthält. Als besonders bevorzugte Substanzen haben sich Substanzen aus der Klasse der Ectoine, insbesondere Ectoin und Hydroxyectoin bewährt.

Des weiteren kommen Verbindungen in Betracht, die an den funktionellen Gruppen der genannten Ectoinverbindungen derivatisiert sind. Im Falle des Hydroxyectoins können beispielsweise aus der Hydroxylgruppe Ether oder Ester gebildet werden. Aus der Carboxylgruppe können Amide oder Ester gebildet werden. Die Carboxylatgruppe kann ersetzt werden durch eine Carbonylgruppe oder eine Sulfonsäuregruppe bzw. deren Ester und Amide.

Als extrahierbare Naturstoffe aus Bakterienkulturen kommen insbesondere Osmotika der folgenden Gruppen in Betracht: Ectoine, insbesondere Ectoin und Hydroxyectoin, die als hygroskopische Verbindungen eingesetzt werden kennen; Aminosäuren und ihre Derivate, insbesondere N-Acetylierte Diaminosäuren, wie N-Acetyl-Lysin und N-Acetyl-Omithin, N-modifizierte Diccarboxamide; Zucker und ihre Derivate, beispielsweise.Trehalose (Trockenstabilisator) , Polyole, Betaine, Peptide und Proteine.

Insbesondere wird von Halomonas efongata halophilus und Marinococcus das optisch aktive Hydroxyectoin der nachstehenden Formel gebildet.

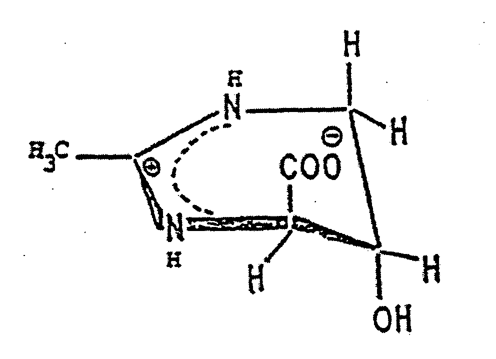

### S,S-α-Amino-β-hydroxyectoin

Diese Substanz ist beispielsweise neben anderen Ectoin-Derivaten zur Kryoprotektion von biologischen Wirkstoffen, Zellen, Blutkonserven usw.. einsetzbar und kann in Medizintechnik, Kosmetik und pharmazeutischer Industrie Verwendung finden.

Ectoin und Ectoin-Derivate wie Hydroxyectoin können auch in Arzneimitteln verwendet werden. Insbesondere kann Hydroxyectoin zur Herstellung eines Arzneimittels zur Behandlung von Hauterkrankungen eingesetzt werden. Andere Einsatzgebiete des Hydroxyectoins und anderer Ectoin-Derivate liegen typischerweise in Gebieten in denen z .B. Trehalose als Zusatzstoff verwendet wird. So können Ectoin-Derivate, wie Hydroxyectoin, als Schutzstoff in getrockneten Hefe- und Bakterienzellen Verwendung finden. Auch pharmazeutische Produkte wie nicht glykosylierte, pharmazeutisch wirksame Peptide und Proteine z.B. t-PA können mit Ectoin oder seinen Derivaten geschützt werden.

Ein erster Gegenstand der vorliegenden Erfindung ist daher die Verwendung von Hydroxyectoin und/oder Ectoin als wirksamer Zusatz in der Kryoprotektion von Zellen in der kosmetischen Industrie.

Ein zweiter Gegenstand der vorliegenden Erfindung ist die Verwendung von (S)-1 ,4,5,6-Tetrahydro-2-methyl-4-pyrimidin-carbonsäure (Ectoin) oder (S,S)-1,4,5,6-Tetrahydro-5-hydroxy-2-methyl-4-pyrimidin-carbonsäure zur Herstellung einer kosmetischen Zubereitung.

Ein weiterer Gegenstand der vorliegenden Erfindung sind kosmetische Zubereitungen enthaltend (S-1,4,5,6-Tetrahydro-2-methyl-4-pyrimidincarbonsäure (Ectoin) oder (S,S)-1 ,4,5,6-Tetrahydro-5-hydroxy-2-methyl-4-pyrimidin-carbonsäure mit der Maßgabe, dass Zubereitungen ausgeschlossen sind, die aus Ectoin oder Hydroxyectoin bestehen, die aus Ectoin oder Hydroxyectoin und Wasser bestehen, oder die Wasser, Ectoin oder Hydroxyectoin und ein Enzym ausgewählt aus Lactatdehydrogenase und Phosphofructokinase enthalten.

Das Verfahren zur in vivo Gewinnung von Inhaltsstoffen aus Zellen geht davon aus, das vorkultivierte Zellen, die in einem bestimmten ersten Zustand gewachsen sind, in einen zweiten Zustand überführt werden. Durch diesen Schock werden die Zellen gezwungen, durch Abgabe von Zellinhaltsstoffen in die Umgebung zu reagieren, um eine Anpassung der Zellen an die Bedingungen, die im zweiten Zustand herrschen, zu erreichen. Dabei müssen die vorgenannten Verfahrensschritte mindestens zweimal durchlaufen werden (Wechselschock) .

Die Veränderung in der Umgebung der Zellen im ersten Zustand kann beispielsweise durch Veränderung des Druckes, der Temperatur, der Konzentration von Stoffen, dem pH-Wert oder Kombinationen dieser Maßnahmen erzielt werden.

So ist es beispielsweise möglich, einen Schock durch Änderungen der osmotischen Bedingungen im Kulturmedium auszulösen.

Das Verfahren kam vorzugsweise mit weiteren Maßnahmen kombiniert werden, wie solchen, die eine Erhöhung der Permeabilität von Zellmembranen hervorrufen, wie beispielsweise Elektroporationsverfahren. Die Permeabilität der Zellmembranen kann auch durch permeabilisierende Substanzen verändert werden. Dazu gehören insbesondere porenbildende und/oder diffusionsfordernde Peptid- und Depsipeptidantibiotika, z .B . Valinomycin,

Polymyxin, Gramicidin, Nisin sowie Detergenzien wie z..B. Natriumdodecylsulfat, Triton X, Cetyltrimethylamoniumbromid. Auch Wachstum der Zellen in Mangelmedien kann zu einer Erhöhung der Zellpermeabilität führen. Dies geschieht insbesondere dann, wenn den Zellen bestimmte Nährstoffe, wie Biotin oder Mangan, vorenthalten werden. Auch durch Verwendung von Mutanten, deren Lipidstoffwechsel gestört ist, kann die Zellmembran der betreffenden Zellen durchlässiger sein.

Aufgrund der Tatsache, dass die Zellen nicht zerstört werden, sondern lediglich durch Leckagen, die in Form von spezifischen oder unspezifischen Poren, oder aktiven Transportsystemen vorliegen können, Zellinhaltsstoffe ins Medium abgeben, kann durch Regeneration des ersten Zustandes und erneuten Wechsel in den zweiten Zustand das erfindungsgemäße Verfahren kontinuierlich gestaltet werden. Dies erfolgt erfindungsgemäß in Form von sogenannten Wechselschocks in zyklischer Prozessführung. Vorzugsweise werden zwischen den Wechselschocks die Zellen solange bei konstanten Bedingungen gehalten, bis zwischen Zellen und Umgebung quasi-stationäre Bedingungen vorliegen.

Als Zellen, die dem erfindungsgemäßen Verfahren unterworfen werden können, kommen Mikroorganismen, insbesondere Bakterien, Pilze, Hefen, Algen, Rotalgen oder Zellen höherer Organismen, tierische oder pflanzliche Gewebe, oder Organe in Betracht.

In bestimmten Konstellationen kann es vorteilhaft sein, als Zellen genetisch manipulierte Zellen einzusetzen, die bestimmte Stoffe synthetisieren und diese in Folge des Schocks in die Umgebung sezernieren.

Nach dem Verfahren arbeitet man in einer bevorzugten Ausführungsform, wie im folgenden beschrieben. Beispielsweise produzieren halotolerante Eubakterien in Anpassung an hohe Salinität niedermolekulare Naturstoffe (Osmotika), die native Enzyme in einem Milieu geringer Wasseraktivität stabilisieren. Sie können als Schutzstoffe in der Enzymtechnologie ein gesetzt werden, und zwar als Hitze-, Gefrier- und Trocknungsschutzsubstanzen. Viele dieser "Solute", wie beispielsweise Verbindungen aus der Klasse der Tetrahydropyrimidincarbonsäuren, sind biotechnologisch in Anwendung des Verfahrens leicht zugänglich. Das Verfahren nutzt die Fähigkeit der halotoleranten Organismen, bei einem Verdünnungsstress rasch große Mengen organischer Osmotika zu exportieren, um osmotisch bedingtes Platzen der Zellen zu vermeiden. Vorteilhaft am Verfahren ist die Tatsache, dass die Ausscheidung der zytoplasmatisch akkumulierten Substanzen auch bei Wildstämmen induzierbar ist, und dass das Produkt in relativ reiner Form in einem verdünnten wässrigen Medium vorliegt. Die problematische Reinigung niedermolekularer organischer Verbindungen wird dadurch wesentlich vereinfacht. Da die Produktion beispielsweise der Solute vorrangig und unabhängig vom Zellwachstum erfolgt, lässt sich die in-vivo-Extraktion, in Verkörperung des Verfahrens, beliebig oft wiederholen und kann in einem kontinuierlichen Verfahren der Biokonversion mit Biomasserecycling weitergeführt werden.

In einer bevorzugten Ausführungsform des Verfahren werden Mikroorganismen, wie Proteobakterien, insbesondere Halomonadaceae, oder Firmicutes, z.B. Micrococci, eingesetzt.

Durch stufenweises Verdünnen einer dichten Zellkultur, Analyse der ausgeschiedenen Zellinhaltsstoffe und abschließender Lebendzellzahlbestimmung kann ein geeigneter Organismus identifiziert werden, der auch extremen Verdünnungsstress toleriert.

Die Zellen werden vorzugsweise von einer Umgebung mit einer Osmolarität von bis zu 30 Osmol in eine Umgebung möglichst geringer Osmolarität überführt. Die Osmolarität kann auch zwischen 5 und 10 Osmol geändert werden.

Ein bevorzugter Organismus sollte auch ohne komplexe Nährstoffe schnell wachsen, das gewünschte Produkt gegebenenfalls aufgrund genetischer Manipulation in hoher Konzentration im Zytoplasma anreichern können (bis zu 2 M) und Wechselschocks, vorzugsweise zwischen 20 %iger Natriumchloridlösung und reinem Wasser, unbeschadet überstehen. Durch die Inkubation in niederosmolarem Nährmedium (idealerweise Wasser) werden die Zellen zur Abgabe ihrer Osmotika gezwungen. Das von den Zellen getrennte niederosmolare Medium enthält die Osmotika (Produktlösung) . Die zyklische Prozessführung ermöglicht eine wirtschaftlichere Verfahrensführung, da die einmal kultivierten Zellen mehrfach zur Produktion der Osmotika eingesetzt werden können. Ein Aufschluss der Zellen entfällt ebenso wie die Abtrennung von Produkt und Zelltrümmern.

Die zum Austausch der Nährmedien erforderliche Abtrennung von Zellen vom Außenmedium kann auf verschiedene Weise erfolgen. Darunter sind Methoden zu nennen wie Zentrifugation, Filtration, Ausflockung oder Immobilisierung der Zellen. Die Veränderung der Osmolarität kann aber auch durch eine veränderliche Löslichkeit der Osmotika im Außenmedium, z. B. durch Temperatur, durch deren Polymerisationsgrad oder Komplexierung erreicht werden. Entsprechend der Wahl des Trennungsverfahrens weist der Anlagenaufbau zur Kultivierung der Zellen bestimmte Unterschiede auf. Vorteilhaft ist eine hohe Beladung der Anlage mit Zellen, da auf diese Weise die Ausbeute gesteigert werden kann. Die hohe Zelldichte kann z. B. durch eine Hochzelldichtefermentation oder ein Immobilisierungsverfahren, das zu einem hohen Zellanteil im Immobilisat führt, erreicht werden.

Ein typischer Verfahrensverlauf am Beispiel der Kultivierung von Halomonas elongata wird im folgenden beschrieben.

Die Vorkultur von Halomonas elongata wird im Bioreaktor im Batchverfahren angezüchtet. Die Batchfermentation wird solange durchgeführt bis ein Großteil der Kohlenstoffquelle, in Form von beispielsweise Glucose, verbraucht ist. Viele wichtige Nährlösungsbestandteile, wie z. B. Glucose oder Methanol, lassen sich nur in begrenzter Menge einsetzen, da sie in zu großer Konzentration wiederum das Wachstum der Mikroorganismen hemmen. Um dennoch hohe Zelldichten zu erreichen, kann man solche das Wachstum limitierende Stoffe während der gerammten Fermentationsdauer kontinuierlich zufüttern ("Fed-Batch-Kultur"). Erfolgt das Zufüttern mit exponentiell ansteigender Fütterungsrate, so wird das exponentielle Wachstum der Mikroorganismen optimal unterstützt und es wird eine maximale Raum-Zeit-Ausbeute erreicht. Wählt man die Parameter richtig, so kann zu jedem Zeitpunkt des exponentiellen Wachstums die entsprechende Substratmenge zugefüttert werden. Werden die Parameter nicht optimal abgeglichen, kann im Extremfall Substratakkumulationen im Reaktor auftreten und damit das Wachstum der Mikroorganismen gehemmt werden, bis hin zum Absterben der Mikroorganismen.

Die optimale Futterungsstrategie kann beispielsweise nach dem Algorithmus von Keller und Dunn erfolgen (Keller R. and Dunn I.J. Fedbatch microbial culture: models, errors and applications. J. appl. Chem. Biotechnol., 28: 508 bis 514, (1978)).

An die Hochzelldichtefermentation schließt sich der Verfahrensschritt der Konzentrierung der Zellsuspension an. Um anschließend die Solute aus den Zellen zu extrahieren, ist es empfehlenswert, zunächst den Großteil des Außenmediums zu entfernen. Diese Konzentrierung der Zellsuspension kann insbesondere durch Querstromfiltration durchgeführt werden.

Dazu wird die Zellsuspension mittels einer Fördereinrichtung, wie einer Pumpe, durch eine Filtriereinrichtung, beispielsweise eine Filterkassette, gefordert. Querstromfiltrationen finden seit einigen Jahren vielfältige Anwendung, z. B.:
- in der Enzymgewinnung aus Zellhomogenisaten
- zur Zellernte sowohl von Bakterien - als auch von Tierzellkulturen
- in Fermentationen mit Zellrückhaltung zur Steigerung der Zelldichte (Katalysatprdichte) und bei gleichzeitiger Abführung des (inhibierenden) Produkts im Filtrat.

Nach der Konzentrierung der Zellen werden diese einem hypoosmotischen Schock ausgesetzt. Der Verdünnungsstress wird beispielsweise durch Zugabe von sterilem Aqua destillata bis zum Ausgangsvolumen der Kulturflüssigkeit herbeigeführt. Vorzugsweise wird das Wasser innerhalb eines Zeitraumes von weniger als 30 Minuten zugegeben. Daraufhin erfolgt vorzugsweise eine zweite Konzentrierung der Zellsuspension, die analog zur ersten Filtration durchgeführt werden kann. Das nunmehr abfiltrierte Außenmedium enthält das Produkt und wird aufgefangen. Die Produktlösung kann dann nach an sich bekannten Verfahren aufgearbeitet werden. Daran anschließend wird vorzugsweise bei kontinuierlicher Verfahrensführung ein hyperosmotischer Schock ausgeübt, indem eine hochkonzentrierte Nährstofflösung mit hohem Salzgehalt wiederum bis zum Ausgangsvolumen zugegeben wird. Es kann sich empfehlen, die Zugabe in einem längeren Zeitintervall durchzuführen als bei Ausführung des hypoosmotischen Schocks.

Es kann vorteilhaft sein, nach dem hyperosmotischen Schock die Zellen in einer Regenerationsphase zu halten, um eine vollständige Synthese der kompatiblen Solute im Zellinnern zu erleichtern. Danach ist die Zellkulturlösung zu einem weiteren Zyklus bereit, der aus den Schritten der Konzentrierung der Zellen, dem hypoosmotischen Schock, Filtration der Produktlösung, hyperosmotischen Schock und der Regenerationsphase besteht.

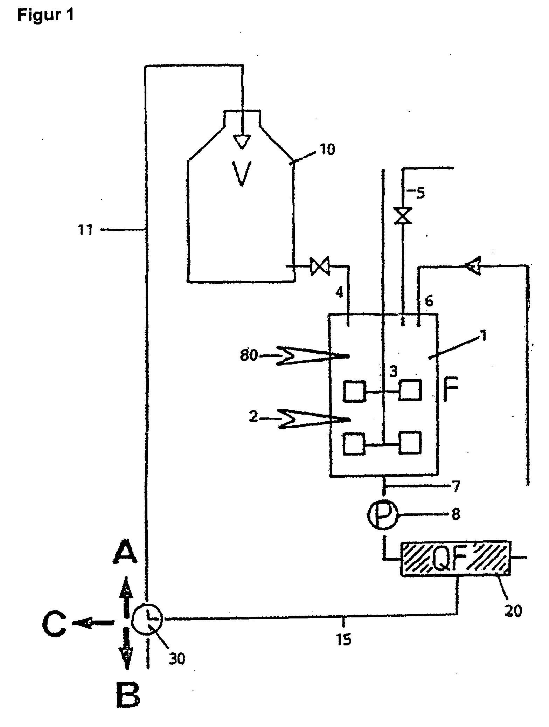

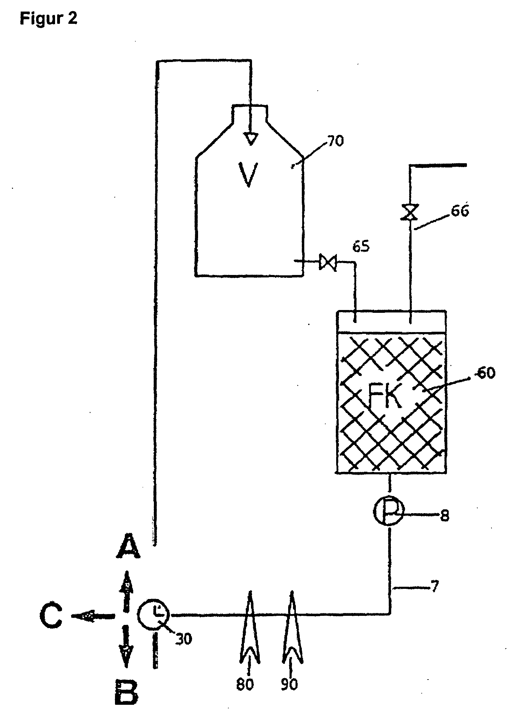

Die Figuren 1 und 2 zeigen schematische Darstellungen der Prozessführung im Filtrationsmodus (Figur 1) und im Festkörperverfahren (Figur 2).

Figur 1 beschreibt eine Anordnung bestehend aus einem Fermenter 1, in dem die Bakterienkultur gezüchtet wird. Der Fermenter ist mit einem Trübungsmesssystem 2, einem Leitfähigkeitsmesssystem 80, einem Rührer 3 und den Zu- und Ableitungen 4, 5, 6 und 7 versehen. Das Vorratsgefäß 10 steht über die Zuleitung 4 mit dem Fermenter in Verbindung und weist vorzugsweise darüber hinaus eine Verbindung 11 zu der Querstromfiltrationseinheit 20 über ein Mehr-Wege-, insbesondere 3-Wege-Ventil, auf.

Über die Pumpe 8 kann das Medium durch die Querstromfiltrationseinheit 20 abgenommen und der Reaktorinhalt konzentriert werden. Über die Leitung 15 kann dann das abgezogene Medium über das 3-Wege-Ventil 30 in den Vorratsbehälter 10 überführt (Stellung A) oder aufgefangen und gegebenenfalls nach Aufarbeitung (Reinigung) wiederverwendet werden (Stellung C) . Über die Zuleitung 5 wird danach der hypoosmotische Schock ausgelöst, indem Wasser zugeleitet wird bis das ursprüngliche Volumen im Reaktionsgefäß 1 wieder hergestellt ist. Danach kann erneut über die Ableitung 7 und die Querstromsfiltrationseinheit 20, das nunmehr osmotisch verdünnte Kulturmedium entfernt werden und dann über eine alternative Schaltung des 3-Wege-Ventils 30 (Stellung B) zur Produktseite in ein entsprechendes Auffanggefäß 40 überführt werden.

Die ursprünglichen osmotischen Verhältnisse können durch Zugabe des Mediums aus dem Vorratsbehälter 10 bzw. hochsalines Medium wiederhergestellt werden.

In einer anderen Ausführungsform, die in Figur 2 dargestellt ist, ist die Festkörper- oder Wirbelschichtkultur 60 mit den Zuleitungen 65, 66 ausgestattet. Die Kultur 60 besteht aus den entsprechenden immobilisierten Organismen. Die Zuleitung 65 ist mit einem Vorratsgefäß 70 verbunden, in dem die Nährlösung bevorratet werden kann. Über die Zuleitung 66 kann das Medium für den hypoosmotischen Schock zugeleitet werden.

Über die Pumpe 8 kann das Medium jeweils abgezogen werden und je nach Verfahrenschritt entweder über das 3 -Wege-Ventil 30 in das Vorratsgefäß 70 zurückgeführt, aufgefangen und aufgearbeitet werden (Stellung C) oder in Stellung B des 3-Wege-Ventils die Produktlösung aufgefangen werden. Vorzugsweise sind in der Ableitung 7 Messeinrichtungen wie Leitfähigkeitsmesssysteme 80 oder Durchflussphotometer 90 zur Kontrolle der Salinität bzw. Produktmonitoring angeordnet.

Ebenfalls können weitere Messsonden, -fühler angeordnet sein. Optional sind jeweils auch Steuereinheiten anschließbar.

### Versuchsbeispiel :

**1,5-I-Bioreaktor**: Biostat M (B.. Braun, Melsungen) mit Messaufsatz HE6. Im Bioreaktor wurden über eine große Zahl von Sonden Messdaten erfasst: pH-Sonde, pO₂-Sonde, OD-Sonde, Schaumsonde, Temperatursonde und Leitfähigkeitssonde. Die Messdaten werden zum zweiten wesentlichen Bestandteil der Anlage, dem Fermenterleitsystem, weitergeleitet. Fermenterleitsystem: (Münzer & Diehl, Overath), bestehend aus einem Batch-Controller (zur Datenerfassung und -digitalisierung sowie zur Steuerung und Regulation von Prozessgrößen) und einem IBM-Industriecomputer AT 286 mit FAS-(Fermenter Application-Software)-Programm (zur Visualisierung und Speicherung der Daten sowie zur Programmierung des Batchcontrollers).

**Querstromfiltration**: Querstromfiltrationseinheit Minisette Sanitary Cell System mit Filtrationskassette (SS994C02, Porengröße 0,16 µm, Filterfläche 700 cm², FILTRON, Northborough, MA, U.S.A.).

In diesem Verfahren dient die Querstromfiltration der Konzentrierung der Bakteriensuspension im Fermenter. Der Druck in der Querstromeinheit wird mit Hilfe von Manometern kontrolliert und durch Ventile reguliert.

### Weitere Geräte:

- Trubungsmessgerät AS 82 und Trübungsmesssonde AF 44 S (IMA, Neu-Isenburg-Zeppelinheim) nach dem Prinzip der Streulichtmessung
- Leitfähigkeitsmessgerät LF 537 und Leitfähigkeitsmesssonde Tetracon 96 (WTW, Weilheim)
- Schlauchpumpe 2006 (VERDER, Düsseldorf)
- Schlauchpumpe MV-CA 4 (ISMATEC, Glattbrugg, CH)
- 2 Membranpumpen FE 211 (B. Braun, Melsungen).

**Lösungen**: Jede Fermentation wurde mit 200 ml Vorkultur und 1000 ml WM15 gestartet (verändertes VREELAND-Medium WM15, modifiziert: Nacl (145,8 g), KCI (1,O g), MgSO₄ · 7H₂O (6,5 g), NH₄CL (3,0 g), K₂HPO₄ (0,5 g), CaCl₂ · 2 H₂O (0,01 g), FeSO₄ · 7 H₂O (0,01 g), Glucose-Monohydrat (11,0 g), TES (1,0 ml), Aqua dest. ad 1000 ml, pH 7,0 (mit 1 N HCl), als Vorkulturmedium mit 6 g/l TRIS und 1 N HCl auf pH 7,5 gepuffert, TES: Spurenelementlösung, Medium Nr. 141, DSM 1989).

Zur Regulation des pH-Wertes während der versuche wurden NaOH und HCl eingesetzt. Zur Durchführung der Hochzelldichtefermentation und der Wechselschockschritte wurden FBL-Medium (Glucose-Monohydrat (297,3 g), NH₄Cl (66,9 g), K₂PO₄ (43,5 g), 1 N HCl (100 ml), Auqa dest. ad 500 ml) und KS30-Medium zur Regulation der Salinität (NaCl (300 g), KCI (2 g), MgSO₄ · 7 H₂o (13 g), CaCl₂· 2 H₂O (0,02 g), FeSO₄ · 7 H₂O (0,02 g), TES (2 ml), Aqua dest. ad 1000 ml, pH 7) sowie Aqua dest. benötigt.

**Batch-Kultur**: Der erste Verfahrensschritt war die Anzucht von Halomonas elongata im Bioreaktor im Batchverfahren. Die Fermentationsbedingungen während der gesammten Fermentation waren: pH 7.5, 25°C, Belüftungsrate 0.16 I/min Luft, 15 % NaCl. Die Batchfermentation wurde solange durchgeführt, bis ein Großteil der Glucose verbraucht war.

Die **Fütterungsstrategie** kann nach Keller & Dünn ermittelt werden.

Für die auftretenden Parameter Startbiomassekonzentration (Xₒ), Startvolumen (Vₒ); Wachstumsrate, Ertragskoeffizient und Substratkonzentration müssen die geeigneten Werte gesucht werden. Die Startparameter Biomassekonzentration (Xₒ) und Startvolumen (Vₒ) wurden am Ende der Batchphase ermittelt. Der Ertragskoeffizient Yₓₛ beträgt beim Wachstum von Halomonas elongata in Glucose/Mineralsalz-Medium 0,5. Die Substratkonzentration Sᵢₙ in der FBL betrug 540 g/l; die Substratkonzentration S im Fermenter war am Ende der Batchphase nahe 0 und sollte durch die Fütterungsstrategie nicht groß werden. Deshalb wurde S für die Berechnung der Fütterungsrate vernachlässigt. Der letzte Startparameter der Fütterungsstrategie, die Wachstumsrate, wurde empirisch bestimmt.

Die Fütterungsstrategie wurde in das FAS programmiert und die Fütterungsrate mit Hilfe eines ebenfalls programmierten Zeittaktes für jeden Zeitpunkt der Hochzelldichtefermentation berechnet. Über den Batchcontroller wurde die Fütterungsrate an eine Membranpumpe übergeben und die Fedbatchlösung mit der entsprechenden Flussrate in den Fermenter gepumpt. Es wurden Zelldichten bis zu 120 g/l Frischgewicht (entspricht 40 g/l Trockengewicht) erreicht.

**Konzentrierung der Zellsuspension**: Um anschließend die Solute aus den Zellen zu extrahieren, musste ein Großteil des Außenmediums entfernt werden. Diese Konzentrierung der Zellsuspension wurde mit einer Querstromfiltrationsanlage durchgeführt. Dazu wurden die Zellsuspension mit einer Schlauchpumpe durch die Filterkassette gepumpt. Der Druck in der Filterkassette wurde so reguliert, dass er am Ausgang des Filtermoduls um etwa 0,5 bar geringer als im Eingang war. Er durfte jedoch 3 bar nicht übersteigen. Die Filtrationsdauer hing vom Zustand der Filterkassette ab und schwankte zwischen 30 min bei neuen Kassetten und 2 h bei älteren. Für die Dauer der Filtration wurde die Drehzahl des Rührers im Fermenter auf 300 UpM reduziert und die Zuluft vollständig abgestellt, um ein Eindringen von Luftblasen in den Filter zu verhindern. Das Volumen der Zellsuspension, wurde durch die Filtration auf 1/5 des Ausgangsvolumens reduziert (dies entspricht einer Zelldichte von bis zu 600 g/l Frischgewicht) .

**Hypoosmotischer Schock**: Der Verdünnungsstress wurde durch die Zugabe von sterilem Aqua dest. bis zum Ausgangsvolumen herbeigeführt. Das Wasser wurde während eines Zeitraums von 10 bis 30 min zugegeben.

**Filtration der Produktlösung**: Die zweite Konzentrierung der Zellsuspension wurde durchgeführt, um die Produktlösung von den Zellen abzutrennen. Sie verlief analog zu der ersten Filtration, mit dem Unterschied, dass das abfiltrierte Außenmedium nun das Produkt enthielt. Es konnte eine Konzentration von bis zu 3,7 g Ectoin/l in der Produktlösung erzielt werden.

Hyperosmotischer Schock: Der Salzstress wurde durch die Zugabe von WM18 (Glucose/Mineralsalzmedium mit 18 % (w/v) Salz) bis zum Ausgangsvolumen ausgelöst. Die Zugabe erfolgte über einen Zeitraum von 2 h mit konstanter Flussrate, woraus sich ein zeitlicher Gradient in der Salzkonzentration des Mediums ergibt.

**Regenerationsphase**: Im Anschluss an den hyperosmotischen Schock folgte eine Regenerationsphase von etwa 24 h zur vollständigen Synthese der kompatiblen Solute. Während dieser Zeit wurde mit einer konstanten Flussrate Fedbatchlösung zugefüttert, so dass 1,3 g Glucose pro Stunde zugefüttert wurden.

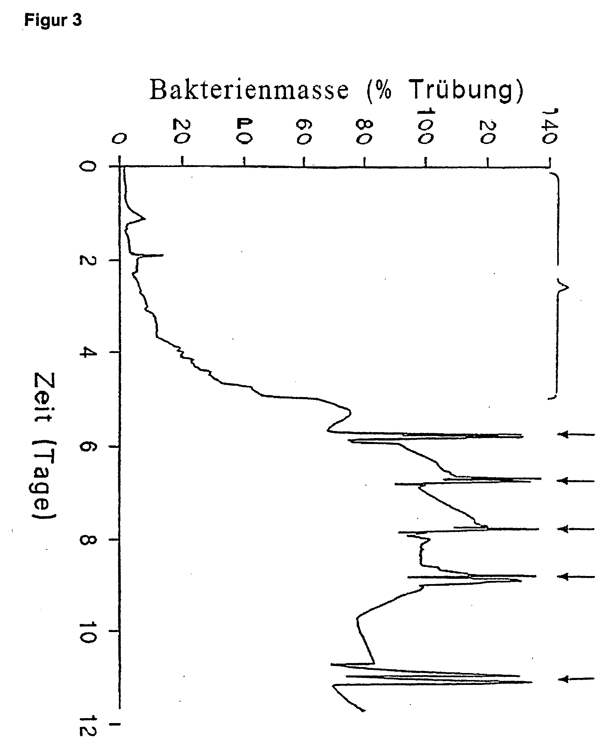

**Wechselschockzyklen**: Nach der Regenerationsphase konnte erneut mit den osmotischen Wechselschocks begonnen werden. Figur 3 zeigt einen typischen Verfahrensverlauf, bei dem 5 Wechselschocks durchgeführt worden sind. Die Zeitpunkte der einzelnen Wechselschocks sind durch die in der Figur angegebenen Pfeile gekennzeichnet. In Figur 3 sind auf der Abszisse die Zeit in Tagen und auf der Ordinate die Bakterienmasse in % Trübung angegeben. Es wurden Halomonas elongata Zellen in einer Hochzelldichtefermentation kultiviert.

## Patentansprüche

1. Kosmetische Zubereitung enthaltend (S)-1,4,5,6-Tetrahydro-2-methyl-4-pyrimidincarbonsäure (Ectoin) oder (S,S)-1,4,5,6-Tetrahydro-5-hydroxy-2-methyl-4-pyrimidincarbonsäure mit der Maßgabe, dass solche Zubereitungen ausgeschlossen sind
• die aus Ectoin oder Hydroxyectoin bestehen,
• die aus Ectoin oder Hydroxyectoin und Wasser bestehen oder
• die Wasser, Ectoin oder Hydroxyectoin und ein Enzym ausgewählt aus Lactatdehydrogenase und Phosphofructokinase enthalten.
